# EUROPEAN PATENT SPECIFICATION

(11) **EP 3 600 211 B1**
(45) Date of publication and mention of the grant of the patent: **27.10.2021**
(21) Application number: 18709798.5
(22) Date of filing: 28.02.2018
(51) Int. Cl.: A61J 1/20, A61M 39/20

(54) **REDUCER FOR A SYRINGE**
REDUZIERSTÜCK FÜR EINE SPRITZE
RÉDUCTEUR POUR UNE SERINGUE

(30) Priority: 23.03.2017 IT 201700032091
(43) Date of publication of application: 05.02.2020
(73) Proprietor: Bormioli Pharma S.P.A., 20123 Milano (IT)
(72) Inventor: PAGANUZZI, Valerio, 43126 Parma (IT)
(74) Representative: Monelli, Alberto
(86) International application number: PCT/IB2018/051284
(87) International publication number: WO 2018/172868

(56) References cited:
- WO-A1-2012/170813
- WO-A1-2016/166197
- US-A1- 2016 038 374
- US-A1- 2016 213 573

## Description

### Technical field

The present invention relates to a reducer applicable to an opening of a container.

Such reducer defines a concavity adapted to house a portion of a syringe doser.

### Background art

Typically a syringe doser is used to facilitate the administration of pharmaceutical products by mouth to a child. On the bottom of the concavity there is a hole so as to allow the product placed in the container to be collected through the syringe doser. The syringe doser comprises a piston and a hollow body that acts as a guide for the piston and that houses the product collected.

Once the product has been collected, the syringe doser is removed from the concavity and partially introduced into the child's mouth.

A drawback of this construction solution is connected with the fact that during the collection of the product a depression is generated in the container. This attracts external air into the hollow body of the syringe doser through an annular seal between a piston and an internal cylindrical wall of the hollow body along which the piston moves; therefore the air enters not from the head end of the hollow body (where there is a hole for collecting the product) but from the opposite end of the hollow body. This means that inside the syringe doser, together with the collected product, there is an undesired excess of air.

A syringe adapter is disclosed by US2016/0038374 and a closure for use with a specimen collection container is disclosed by WO2012/170813.

### Disclosure of the invention

The object of the present invention, as defined by the claims, is to provide a reducer that allows the excess air inside the syringe doser to be removed.

The defined technical task and the specified aims are substantially achieved by a reducer comprising the technical characteristics set forth in one or more of the appended claims.

### Brief description of drawings

Further characteristics and advantages of the present invention will become more apparent from the following indicative and therefore nonlimiting description of a reducer as illustrated in the appended drawings, in which:
- figure 1 shows a perspective view of a reducer according to the present invention;
- figure 2 shows a partially sectional view of the reducer of figure 1;
- figure 3 shows the reducer according to the present invention connected to a syringe doser;
- figure 4 shows a sectional view of an alternative solution of a reducer according to the present invention.

### Detailed description of preferred embodiments of the invention

In the accompanying figures, reference number 1 denotes a reducer applicable to an opening of a container.

Typically such container contains a liquid or viscous product, preferably for pharmaceutical applications. In fact, the reducer 1 is advantageously associated with containers of products intended for children, for example syrups. On this point, the reducer 1 comprises a perimeter annular groove 6 for coupling with a mouth of the container. Such groove 6 is typically circular.

The reducer 1 identifies a seat 2. The seat 2 comprises a bottom 21 and a lateral flank 22 (preferably cylindrical). They define a concavity 20 intended to house a portion of a syringe doser. The bottom 21 has an opening 210 adapted to allow the passage (or rather the collection through the syringe doser) of a fluid present in the container. In fact, the opening 210 allows the inlet of the fluid to be dispensed into the seat 2 for collection by the syringe doser.

The reducer 1 further comprises an annular fluid dynamic sealing structure placed on the bottom 21 and surrounding the opening 210. In particular, the annular structure is in a single body with the remaining parts of the bottom 21. The reducer 1 is a single monolithic body.

Appropriately, the annular groove 6 surrounds the concavity 20. It is preferably coaxial with the concavity 20.

The reducer 1 comprises a passage 3 intended to allow the inlet of air into the container. This enables compensation for the depressurisation induced in the container by the aspiration of fluid by the syringe doser.

The passage 3 is outside the concavity 20. The passage 3 allows the reducer 1 to be crossed for placing the inside and the outside of the container in fluid communication. Therefore, the passage 3 does not contribute to defining the seat 2. As can be seen from the drawings, the fluid communication between the passage 3 and the seat 2 is only possible by passing outside the reducer 1.

The passage 3 comprises an inlet section 31 for the entry of air into said passage 3 and an outlet section 32 for the exit of air from said passage 3. The outlet section 32 for the exit of air from said passage 3 is intended to terminate in the container. In other words, the reducer 1 has opposite ends along the axial development thereof; at one of such ends intended to be placed inside the container, the outlet section 32 is located for the exit of the air from the passage 3. Appropriately, the outlet section 32 is closer to an end of the reducer intended to be placed inside the container with respect to an inlet of a channel for the extraction of the product from the container that terminates in the seat 2.

The seat 2 defines an axial insertion/extraction direction 24 of the syringe doser with respect to the concavity 20. A first and a second imaginary plane 41, 42 are defined, orthogonal to said axial direction 24 and passing through a mouth 23 to insert the syringe doser into the concavity 20 and through the outlet section 32 of said passage 3, respectively. The mouth 23 for the insertion of the syringe doser is opposite the bottom 21. The bottom 21 is interposed between the first and the second imaginary plane 41, 42. Appropriately, a cylindrical axis of symmetry 240 of the seat 2 can be identified. The bottom 21 is interposed between the orthogonal projection of the outlet section 32 along the axis 240 and the mouth 23 for inserting the syringe doser.

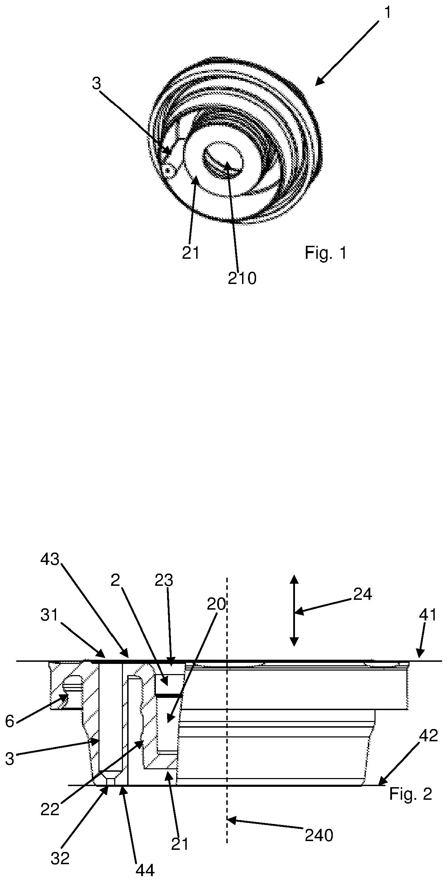

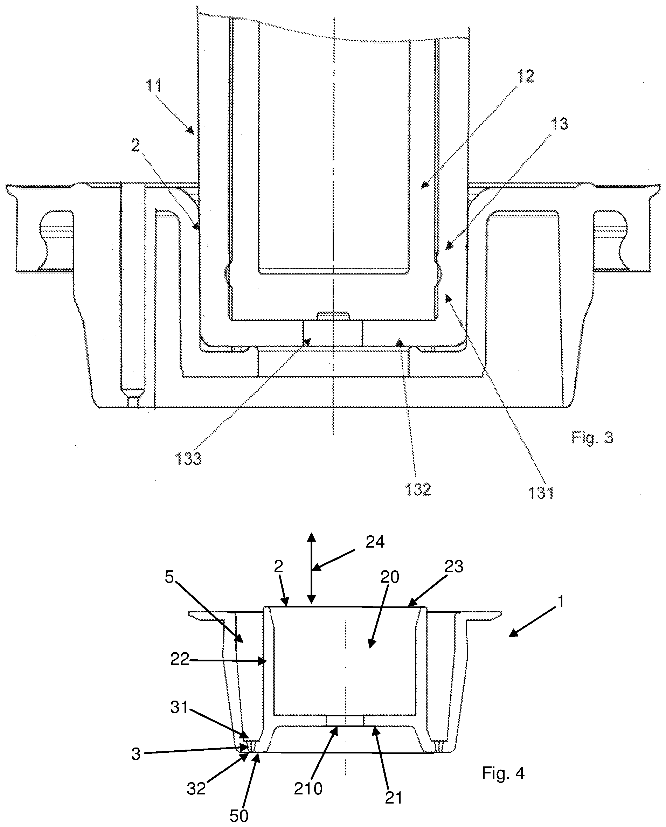

In other words, when the reducer 1 is mounted on a mouth of the container facing upwards, the outlet section 32 of the passage 3 is further down than the bottom 21 (as in figure 2 or 4). In this way, the air that enters into the container through the outlet section 32 is further down than the bottom 21. By overturning the container to bring the fluid to the opening 210 and extract it through the doser, the section 32 is higher up than the bottom 21. The air that enters through the section 32 tends to move upwards, away from the underlying opening 210. In this way, there is no risk of air entering the syringe doser.

The reducer 1 comprises a first and a second annular surface 43, 44 that are opposite one another (facing opposite sides) and between which the reducer 1 extends axially.

As exemplified at least in figures 1 and 2, the inlet section 31 of the passage 3 is obtained in the first annular surface 43 and the outlet section 32 of said passage is obtained in the second annular surface 44.

In figures 1 and 2 the passage 3 is a conduit.

As is instead exemplified in figure 4, the reducer 1 appropriately comprises a recess 5 outside the seat 2. The passage 3 traverses the thickness of a base 50 of said recess 5. In figure 4 the passage 3 is a hole. In that case the inlet and outlet sections 31 and 32 may also be much closer together, or even coinciding.

Preferably, at least one portion of the orthogonal section of said passage 3 has a surface area of less than 2 mm². In this way, sufficiently dense fluids cannot exit the container, even if the same is overturned. In other words, the density of the fluid and the reduced section of the passage 3 prevent the passage of fluid through the passage 3 under the action of the force of gravity.

Appropriately, the passage 3 comprises a narrowing. The aim of this is to minimise the risk of fluid exiting the container through the passage 3. In fact, it is to be noted that the exit of fluid is envisaged through the opening 210 located on the bottom 21.

Appropriately, the passage section of the opening 210 has a surface area that is 4 times the surface area of the minimum crossing section of the passage 3.

The reducer 1 (solution not illustrated) may comprise a valve located along the passage 3. Such valve is movable between an open configuration and a closed configuration. Such valve is normally closed and opens upon the application of a depression greater than a predetermined threshold inside the container. The valve is in a single body with the parts surrounding the reducer 1. As exemplified in the appended figures, the opening 210 cannot, on the other hand, be closed by a valve.

The object of the present invention is also a system comprising:
- a container containing the fluid;
- a reducer 1 having one or more of the characteristics described above. The passage 3 places in fluid communication the inside and the outside of the container by traversing said reducer 1. As indicated above, this allows the depressurisation of the container to be prevented during the dispensing of the fluid contained in the container. In fact, the dispensing of the fluid in the container is accompanied by the entry of air through the passage 3.

The subject matter of the present invention is also a kit as claimed in claim 7.

The syringe doser 11 comprises a piston 12 and a hollow body 13 for guiding said piston 12 intended to house the product collected by the syringe doser 11. The hollow body 13 in turn comprises:
- a lateral wall 131;
- a head end 132 equipped with a hole 133 for collecting the product.

In an operating configuration (for dispensing the fluid contained in the container by means of the doser 11) the lateral wall 131 is in contact with the lateral flank 22 while the head end 132 is abutted by said bottom 21. The method for dispensing the fluid from said container is advantageously as follows:
- overturning the container positioning the reducer 1 connected thereto downwards (relative to the container);
- extracting the piston 12 from the hollow body 13 aspirating the fluid from the container to the hollow body 13 through the opening 210;
- recalling air from the outside of the container to the inside thereof by said passage 3.

Appropriately, the step of recalling air occurs at the same time as the step of extracting the piston 12.

Following the step of recalling air, the air bubbles are directed upwards, away from the reducer 1. In this way, the risk of the air bubbles aspired into the container being aspired into the syringe doser 11 is minimised. The present invention provides important advantages.

Above all, it allows the presence of air in the syringe to be minimised after collecting the product from the container to which the reducer is applied.

In fact, the precautions adopted allow the depression generated by the piston to attract air into the container through the passage 3.

This prevents the depression generated causing the inlet of air through the fluid dynamic seal between the piston and the hollow body.

## Claims

1. A reducer applicable to an opening of a container and identifying a seat (2) comprising a bottom (21) and a lateral flank (22) that define a concavity (20) intended for housing a portion of a syringe doser; said bottom (21) comprising an opening (210) suitable for allowing the passage of a fluid present in the container;
said reducer (1) comprising a passage (3) intended for enabling the air to enter the container to compensate for the depressurisation induced in the container by the aspiration of the fluid by the syringe doser;
said passage (3) being outside said concavity (20), traversing the reducer (1) and being suitable for placing the inside and outside of the container in fluid communication;
said passage (3) comprising an inlet section (31) for the entry of the air into said passage (3) and an outlet section (32) for the exit of the air from said passage (3); said outlet section (32) for the exit of the air from said passage (3) being intended to terminate in the container;
said seat (2) defining an axial insertion/extraction direction (24) for inserting/extracting the syringe doser with respect to said concavity (20); a first and a second imaginary plane (41, 42) are defined that are orthogonal to said axial direction (24) and pass respectively through a mouth (23) to insert the syringe doser into the concavity (20) and the outlet section (32) of said passage (3);
the bottom (21) being interposed between the first and second imaginary plane (41, 42); **characterised in that** the reducer is a single monolithic body.

2. Reducer according to claim 1, **characterised in that** it comprises a first and a second annular surface (43, 44) that are opposite one another and between which the reducer (1) extends; the inlet section (31) of said passage (3) being obtained in said first annular surface (43) and the outlet section (32) of said passage being obtained in said second annular surface (44).

3. Reducer according to any preceding claim, **characterised in that** it comprises a recess (5) outside said seat (2), said passage (3) traversing the thickness of a base (50) of said recess (5).

4. Reducer according to any preceding claim, **characterised in that** at least one portion of said passage (3) has an orthogonal section that has a surface of less than 2 mm².

5. Reducer according to any preceding claim, **characterised in that** said passage (3) comprises a narrowing.

6. A system comprising:
- a container containing the fluid;
- a reducer (1) according to one or more of claims 1 to 5.
said passage (3) placing in fluid communication the inside and the outside of the container by traversing said reducer (1).

7. A kit comprising:
i) a reducer (1) according to one or more of claims 1 to 5;
ii) a syringe doser (11) comprising a piston (12), a hollow body (13) for guiding said piston (12) intended to house the product collected by the syringe doser; said hollow body (13) comprising:
- a lateral wall (131);
- a head end (132) equipped with a hole (133) for collecting a product;
in one operating configuration said lateral wall (131) being in contact with said lateral flank (22), said head end (132) being abutted by said bottom (21).

8. Method of dispensing fluid from a container by the kit of claim 7, **characterised in that** it comprises the following steps:
- overturning the container positioning the reducer (1) connected thereto downwards;
- extracting the piston (12) from the hollow body (13) aspirating the fluid from the container to the hollow body (13) through the opening (210);
- recalling air from the outside of the container to the inside thereof by said passage (3); the bubbles of air inside the fluid that is present in the container moving upwards away from the reducer (1);
the step of recalling air occurring at the same time as the step of extracting the piston (12) from the hollow body (13).

## Patentansprüche

1. Reduzierstück, das an einer Öffnung eines Behälters anbringbar ist und einen Sitz (2) identifiziert, umfassend einen Boden (21) und eine seitliche Flanke (22), die eine Konkavität (20) definieren, die bestimmt ist, einen Abschnitt eines Spritzendosierers aufzunehmen; wobei der Boden (21) eine Öffnung (210) umfasst, die ausgelegt ist, um den Durchgang eines in dem Behälter vorhandenen Fluids zu ermöglichen;
wobei das Reduzierstück (1) einen Durchgang (3) umfasst, der bestimmt ist, um der Luft zu ermöglichen, in den Behälter einzutreten, um den Druckabbau zu kompensieren, der in dem Behälter durch das Ansaugen des Fluids durch den Spritzendosierer hervorgerufen wird;
wobei der Durchgang (3) außerhalb der Konkavität (20) liegt, das Reduzierstück (1) durchquert und ausgelegt ist, um das Innere und das Äußere des Behälters in Fluidkommunikation zu bringen;
wobei der Durchgang (3) einen Einlassabschnitt (31) für den Eintritt der Luft in den Durchgang (3) und einen Auslassabschnitt (32) für den Austritt der Luft aus dem Durchgang (3) umfasst; wobei der Auslassabschnitt (32) für den Austritt der Luft aus dem Durchgang (3) bestimmt ist, in dem Behälter zu enden;
wobei der Sitz (2) eine axiale Einführ-/Ausziehrichtung (24) zum Einführen/Ausziehen des Spritzendosierers in Bezug auf die Konkavität (20) definiert; eine erste und eine zweite imaginäre Ebene (41, 42) sind definiert, die orthogonal zu der axialen Richtung (24) sind und jeweils durch eine Mündung (23) verlaufen, um den Spritzendosierer in die Konkavität (20) und den Auslassabschnitt (32) des Durchgangs (3) einzuführen;
wobei der Boden (21) zwischen der ersten und der zweiten imaginären Ebene (41, 42) angeordnet ist; **dadurch gekennzeichnet, dass** es sich beim Reduzierstück um einen einzelnen monolithischen Körper handelt.

2. Reduzierstück nach Anspruch 1, **dadurch gekennzeichnet, dass** es eine erste und eine zweite ringförmige Fläche (43, 44) umfasst, die einander gegenüberliegen und zwischen denen sich das Reduzierstück (1) erstreckt; wobei der Einlassabschnitt (31) des Durchgangs (3) in der ersten ringförmigen Fläche (43) ausgebildet ist und der Auslassabschnitt (32) des Durchgangs in der zweiten ringförmigen Fläche (44) ausgebildet ist.

3. Reduzierstück nach einem der vorhergehenden Ansprüche, **dadurch gekennzeichnet, dass** es eine Aussparung (5) außerhalb des Sitzes (2) umfasst, wobei der Durchgang (3) die Dicke einer Basis (50) der Aussparung (5) durchquert.

4. Reduzierstück nach einem der vorhergehenden Ansprüche, **dadurch gekennzeichnet, dass** mindestens ein Abschnitt des Durchgangs (3) einen orthogonalen Abschnitt mit einer Fläche von weniger als 2 mm² aufweist.

5. Reduzierstück nach einem der vorhergehenden Ansprüche, **dadurch gekennzeichnet, dass** der Durchgang (3) eine Verengung umfasst.

6. System, umfassend:
- einen Behälter, der das Fluid enthält;
- ein Reduzierstück (1) nach einem oder mehreren der Ansprüche 1 bis 5, wobei der Durchgang (3) das Innere und das Äußere des Behälters in Fluidkommunikation bringt, indem er das Reduzierstück (1) durchquert.

7. Kit, umfassend:
i) ein Reduzierstück (1) nach einem oder mehreren der Ansprüche 1 bis 5;
ii) einen Spritzendosierer (11) umfassend einen Kolben (12), einen Hohlkörper (13) zum Führen des Kolbens (12), der das durch den Spritzendosierer gesammelte Produkt aufnehmen soll; wobei der Hohlkörper (13) umfasst:
- eine Seitenwand (131);
- ein Kopfende (132), das mit einem Loch (133) zum Sammeln eines Produkts ausgestattet ist;
wobei in einer Betriebskonfiguration die Seitenwand (131) in Kontakt mit der seitlichen Flanke (22) steht, wobei das Kopfende (132) an dem Boden (21) anliegt.

8. Verfahren zum Abgeben von Fluid aus einem Behälter durch das Kit nach Anspruch 7, **dadurch gekennzeichnet, dass** es die folgenden Schritte umfasst:
- Umkippen des Behälters, indem das damit verbundene Reduzierstück (1) nach unten positioniert wird;
- Ausziehen des Kolbens (12) aus dem Hohlkörper (13), Ansaugen des Fluids vom Behälter zum Hohlkörper (13) durch die Öffnung (210);
- Rückholen von Luft von der Außenseite des Behälters zu dessen Innenseite durch den Durchgang (3); die Luftblasen in dem Fluid, das sich im Behälter befindet, bewegen sich nach oben weg vom Reduzierstück (1);
wobei der Schritt zum Rückholen von Luft gleichzeitig mit dem Schritt zum Ausziehen des Kolbens (12) aus dem Hohlkörper (13) erfolgt.

## Revendications

1. Réducteur applicable à une ouverture d'un récipient et identifiant un siège (2) comprenant un fond (21) et un flanc latéral (22) qui définissent une concavité (20) destinée à loger une partie d'un doseur de seringue ; ledit fond (21) comprenant une ouverture (210) adaptée pour permettre le passage d'un fluide présent dans le récipient ;
ledit réducteur (1) comprenant un passage (3) destiné à permettre à l'air de pénétrer dans le récipient pour compenser la mise hors pression induite dans le récipient par l'aspiration du fluide par le doseur de seringue ;
ledit passage (3) étant à l'extérieur de ladite concavité (20) traversant le réducteur (1) et étant adapté pour mettre en communication fluidique l'intérieur et l'extérieur du récipient ;
ledit passage (3) comprenant une section d'entrée (31) pour l'entrée de l'air dans ledit passage (3) et une section de sortie (32) pour la sortie de l'air dudit passage (3) ; ladite section de sortie (32) pour la sortie de l'air dudit passage (3) étant prévue pour se terminer dans le récipient ;
ledit siège (2) définissant une direction axiale d'introduction/d'extraction (24) pour introduire/extraire le doseur de seringue par rapport à ladite concavité (20) ; un premier et un second plan imaginaire (41, 42) sont définis qui sont orthogonaux à ladite direction axiale (24) et passent respectivement par une bouche (23) pour introduire le doseur de seringue dans la concavité (20) et la section de sortie (32) dudit passage (3) ;
le fond (21) étant interposé entre le premier et le second plan imaginaire (41, 42) ; **caractérisé en ce que** le réducteur est un corps monolithique unique.

2. Réducteur selon la revendication 1, **caractérisé en ce qu'**il comprend une première et une seconde surface annulaire (43, 44) étant opposées l'une à l'autre et entre lesquelles se prolonge le réducteur (1) ; la section d'entrée (31) dudit passage (3) étant obtenue dans ladite première surface annulaire (43) et la section de sortie (32) dudit passage étant obtenue dans ladite seconde surface annulaire (44).

3. Réducteur selon l'une quelconque des revendications précédentes, **caractérisé en ce qu'**il comprend un renfoncement (5) à l'extérieur dudit siège (2), ledit passage (3) traversant l'épaisseur d'une base (50) dudit renfoncement (5).

4. Réducteur selon l'une quelconque des revendications précédentes, **caractérisé en ce qu'**au moins une partie dudit passage (3) comporte une section orthogonale dont la surface est inférieure à 2 mm².

5. Réducteur selon l'une quelconque des revendications précédentes, **caractérisé en ce que** ledit passage (3) comprend un rétrécissement.

6. Système comprenant :
- un récipient contenant le fluide ;
- un réducteur (1) selon une ou plusieurs des revendications 1 à 5,
ledit passage (3) mettant en communication fluidique l'intérieur et l'extérieur du récipient en traversant ledit réducteur (1).

7. Kit comprenant :
i) un réducteur (1) selon une ou plusieurs des revendications 1 à 5 ;
ii) un doseur de seringue (11) comprenant un piston (12), un corps creux (13) servant à guider ledit piston (12) prévu pour loger le produit recueilli par le doseur de seringue ; ledit corps creux (13) comprenant :
- une paroi latérale (131) ;
- une extrémité de tête (132) pourvue d'un trou (133) pour recueillir un produit ;
dans une configuration de fonctionnement, ladite paroi latérale (131) étant en contact avec ledit flanc latéral (22), ladite extrémité de tête (132) venant se mettre en butée contre ledit fond (21).

8. Procédé de distribution de fluide à partir d'un récipient par le kit selon la revendication 7, **caractérisé en ce qu'**il comprend les étapes suivantes :
- retourner le récipient en positionnant le réducteur (1) qui lui est relié vers le bas ;
- extraire le piston (12) du corps creux (13) en aspirant le fluide du récipient vers le corps creux (13) à travers l'ouverture (210) ;
- rappeler l'air de l'extérieur du récipient vers l'intérieur de celui-ci par ledit passage (3) ; les bulles d'air à l'intérieur du fluide étant présent dans le récipient se déplaçant vers le haut en s'éloignant du réducteur (1) ;
l'étape consistant à rappeler l'air se produisant en même temps que l'étape d'extraction du piston (12) du corps creux (13).
